(19) Europäisches Patentamt

European Patent Office

Office européen des brevets

(11) EP 0 667 328 B1

(12) EUROPÄISCHE PATENTSCHRIFT

(45) Veröffentlichungstag und Bekanntmachung des Hinweises auf die Patenterteilung:
16.09.1998 Patentblatt 1998/38

(51) Int. Cl.$^6$: C07C 25/13, C07C 17/23

(21) Anmeldenummer: 95100955.4

(22) Anmeldetag: 25.01.1995

(54) **Verfahren zur Herstellung von aromatischen Fluorverbindungen**

Process for the preparation of aromatic fluoro compounds

Procédé de préparation de composés de fluor aromatiques

(84) Benannte Vertragsstaaten:
BE CH DE ES FR GB IE IT LI NL

(30) Priorität: 11.02.1994 DE 4404343
16.12.1994 DE 4444904

(43) Veröffentlichungstag der Anmeldung:
16.08.1995 Patentblatt 1995/33

(73) Patentinhaber: Clariant GmbH
65929 Frankfurt am Main (DE)

(72) Erfinder:
• Schach, Thomas, Dr.
D-64579 Gernsheim (DE)
• Papenfuhs, Theodor, Dr.
D-60433 Frankfurt am Main (DE)

(56) Entgegenhaltungen:
EP-A- 0 302 326        EP-A- 0 598 338

## Beschreibung

Die vorliegende Erfindung betrifft ein verbessertes Verfahren zur Herstellung von aromatischen Fluorverbindungen respektive von Fluorbenzolen durch katalytische Abspaltung von Halogen aus brom- und/oder chlorhaltigen Fluoraromaten respektive Fluorbenzolen in Form des entsprechenden Halogenwasserstoffs.

Fluoraromaten finden eine breite Anwendung im Bereich des Pflanzenschutzes und als Synthesebausteine in Pharmavorprodukten.

Durch elektrophile und nukleophile Substitution können Aromaten abhängig von ihrem vorhandenen Substitutionsmuster nur an ganz bestimmten Positionen weiter substituiert werden. Bei der Synthese von Aromaten tritt aber oft der Umstand ein, daß gerade an den weniger bevorzugten Positionen ein Substituent eingebracht werden muß. Es existieren nun eine Reihe von Strategien, dieses Problem zu lösen. So lassen sich unerwünschte Positionen im Aromaten mit Substituenten blockieren, die einerseits einfach ins Molekül einzuführen sind und andererseits ebensoeinfach wieder entfernt werden können. Als Substituenten der Wahl bieten sich die Halogene Brom und Chlor an, die sehr einfach durch elektrophile Substitution in ein aromatisches System eingeführt werden, diese Position im Molekül für weitere Angriffe sperren, gegebenenfalls die elektronischen Verhältnisse im Molekül für den Eintritt weiterer Substituenten günstig beeinflussen und deren Abspaltung am Ende der Synthesesequenz einfach möglich ist.

Ein besonders einfacher Weg, Fluor in ein Molekül einzubringen, führt über die entsprechenden Nitroverbindungen, die durch ihre starke Aktivierung für nukleophile Substitutionen hohe Selektivitäten und Ausbeuten in Halexreaktionen ermöglichen. Werden die Nitrofunktionen im Endprodukt nicht mehr benötigt, gilt es, diese am Ende der Synthese zu entfernen. Das klassische Verfahren führt über die Anilinzwischenstufe, die durch reduktive Desaminierung die gewünschten Aromaten liefert. Neben zum Teil aufwendigen verfahrenstechnischen Maßnahmen (beispielsweise gegen Korrosion), werden zumeist nur mäßige Selektivitäten und Ausbeuten erreicht. Daneben lassen sich nur ungünstige Raum/Zeitausbeuten erzielen und hohe Materialkosten werden erforderlich, wenn hypophosphorige Säure als Reduktionsmittel verwendet wird.

Sollen Fluornitroverbindungen als günstige Vorstufen in der Synthese von Fluoraromaten verwendet werden, die keine Stickstoff-Funktion mehr enthalten, bietet es sich an, die Nitrofunktion über die Synthesesequenz denitrierende Chlorierung und anschließende reduktive Entchlorierung wieder zu entfernen.

Neben diesen exemplarischen Beispielen sind noch eine Vielzahl von Möglichkeiten denkbar, in denen die reduktive Halogenabspaltung eingesetzt werden kann. Beispielsweise treten bei der Halogenierung von Fluoraromaten neben den gewünschten Isomeren auch Nebenverbindungen auf, was gerade bei sehr teuren Fluorverbindungen hohe Kosten verursachen kann, falls für diese Verbindungen keine Verwendung besteht. Auch in diesen Fällen kann die reduktive Enthalogenierung als Methode der Wahl verwendet werden, um teure Ausgangsverbindungen durch das Entfernen der Halogenatome wieder zurückzugewinnen.

Gerade bei der Synthese von reinen Fluorbenzolen, kann die reduktive Enthalogenierung als Methode der Wahl eingesetzt werden um auf elegante Weise über günstig herzustellende Chlorfluorverbindungen (Zugänglich, beispielsweise durch denitrierende Chlorierung über die entsprechenden Nitrofluorverbindungen), die entsprechenden Fluorbenzole herzustellen. Die direkte Halex-Synthese, ausgehend von den entsprechenden Chlorverbindungen gelingt im allgemeinen nicht oder nur in sehr schlechten Selektivitäten und Ausbeuten, da unter den benötigten Reaktionstemperaturen und Bedingungen die Endprodukte meist nur kurze Zeit stabil sind (Pews, R. G.; Gall, J. A.; J. Fluorine Chem., 50(3), 371 - 5; EP 371563).

Die EP-A-0 302 326 betrifft ein Verfahren zur Herstellung von mit Fluor und gegebenenfalls zusätzlich mit Chlor substituierten Benzotrifluoriden durch Hydrierung von mit Fluor und Chlor substituierten Benzotrifluoriden in Gegenwart eines Katalysators und eines Halogenwasserstoffakzeptors. Als Halogenwasserstoffakzeptor werden die verschiedensten anorganischen und organischen Basen, beispielsweise der Hydroxide, Carbonate, Acetate und Ammoniumsalze der Alkali- und Erdalkalimetalle und Amine genannt.

Ein alternativer Weg Fluoraromaten herzustellen, führt über den Weg der Amindiazotierung, mit den unterschiedlichen Varianten wie Schiemann- und Balz-Schiemann-Reaktion. (JP 01283230). Die Technik der Amindiazotierung mit HF hat bei einigen wenigen Verbindungen bereits großtechnischen Maßstab erreicht, läßt sich aber nur auf bestimmte Aniline als Startsubstanz anwenden. Gelingt die Desaminierung mit HF nicht, kann der etwas universellere Weg der Zwischenisolierung von Tetrafluoroboraten und deren anschließende Zersetzung zu den entsprechenden Fluoraromaten angewendet werden. Reaktionen dieses Typs sind technisch schwer zu handhaben und verursachen aufgrund niedriger Ausbeuten und Raum-Zeitausbeuten hohe Produktionskosten.

Die reduktive Enthalogenierung ist somit von sehr großer Bedeutung in der Aromatenchemie, insbesondere für die Fluoraromatensynthese. Allerdings treten bei der Durchführung dieser Reaktion eine Reihe verfahrenstechnischer Probleme auf, die bislang nicht zufriedenstellend gelöst werden konnten.

Die Reaktionen werden im allgemeinen in Gegenwart eines Katalysators wie beispielsweise Palladium, eines Lösungsmittels und einer wässrigen Base wie beispielsweise Natronlauge durchgeführt. Reduktive Enthalogenierungen von Chlor-/Brom-Fluorbenzolen liefern unter diesen Reaktionsbedingungen nur mäßige Selektivitäten und Ausbeuten. In der Regel verlaufen diese Reaktionen nur schwer reproduzierbar (Katalysator-Vergiftungen) und die Selektivität wird durch die Abspaltung von Fluor deutlich verschlechtert. Chlorid-Korrosion ist in vielen Fällen nicht zu vermeiden, da für die meisten der Enthalogenierungen Reaktionstemperaturen über 100°C benötigt werden.

Mit der Fluorabspaltung treten zwei in vielen Fällen nicht zu lösende Probleme in den Vordergrund. Zum einen lassen sich die mit unterschiedlichen Fluoridgehalten gebildeten Rohprodukte praktisch nicht oder nur mit einem sehr aufwendigen Trennverfahren reinigen, da ihre Siedepunkte in den meisten Fällen praktisch identisch sind. Zum anderen kann das in der Reaktion gebildete Fluorid zu weiterer Korrosion führen, der nur mit hohen Ansprüchen an das Reaktormaterial begegnet werden kann.

Desweiteren führt die hohe Nukleophilie der verwendeten Base (z.B.: wäßrige NaOH) zur Bildung von Phenolen, wodurch die Selektivität dieser Reaktion weiter verschlechtert wird. Werden die üblichen Amine wie beispielsweise Trimethylamin, Triethylamin verwendet, kann die Bildung von Nebenreaktionen weitestgehend unterdrückt werden. Die gebildeten Salze dieser Basen, bzw. die freie Base selbst lassen sich entweder gar nicht oder nur schwer zurückgewinnen, sodaß neben hohen Kosten eine erhebliche organische Verunreinigung des Abwassers auftritt und eine technische Realisierung des Verfahrens nahezu ausschließt.

In Anbetracht der Vielzahl von Nebenreaktionen und verfahrenstechnischer Probleme der bislang bekannten Herstellungsverfahren, besteht ein großes Bedürfnis nach einer verbesserten Synthesemöglichkeit zur Herstellung von hochreinen Fluorbenzolen, wobei neben guten bis sehr guten Ausbeuten auch leicht zugängliche und im technischen Maßstab zur Verfügung stehende Vorstufen gefordert sind. Die reduktive Chlorabspaltung erweist sich als ein sehr günstiges Herstellungsverfahren, läßt sich aber aufgrund der hohen Korrosion, der ungünstigen Produktqualität und der nur schwer zu reproduzierbaren Versuche (Katalysator-Vergiftungen) bisher technisch kaum umsetzen. Es bestand daher ein großes Bedürfnis, die beschriebenen Mängel zu beseitigen und ein technisch günstiges Verfahren zu entwickeln.

Die Aufgabe wird gelöst durch ein Verfahren zur Herstellung von Fluoraromaten der Formel (I) - ausgenommen 1,3-Difluorbenzol -

$$F_n ArR^1R^2R^3 \qquad (I)$$

worin

Ar Phenyl, Naphthyl, Pyridyl, $R^1$, $R^2$, $R^3$ unabhängig voneinander Wasserstoff, Halogen, $(C_1-C_4)$-Alkyl, Phenyl, $NR_2$, OR, CN, CHO, COR, wobei R Wasserstoff, $(C_1-C_6)$-Alkyl und n = 1, 2, 3, 4 oder 5

bedeuten, dadurch gekennzeichnet, daß man Fluoraromaten der Formel (II)

$$X_m F_n ArR^1R^2R^3 \qquad (II)$$

worin Ar, $R^1$, $R^2$, $R^3$ und n die oben erwähnte Bedeutung besitzen,
X für Chlor- oder Bromatome steht und
m = 1, 2, 3, 4 oder 5 bedeutet, in Gegenwart eines Palladium-Katalysators, eines nicht wasserlöslichen Amins, das nicht wasserlösliche Hydrohalogenide bildet und gegebenenfalls eines inerten Lösungsmittels mit Wasserstoff umsetzt.

Man setzt als geeignete Ausgangsstoffe Fluoraromaten der Formel (II), worin $R^1$, $R^2$ und $R^3$ unabhängig voneinander Wasserstoff, Fluor, Chlor, Brom, $(C_1-C_4)$-Alkyl, Phenyl, $NR_2$, OR, CN, CHO oder COR, insbesondere Wasserstoff, Fluor, $(C_1-C_4)$-Alkyl, OR, CN oder COR, bevorzugt Wasserstoff oder Fluor bedeuten, ein.
Das erfindungsgemäße Verfahren läßt sich mit gutem Erfolg bei der Umsetzung von Fluoraromaten der Formel (II), worin Ar für Pyridyl oder Phenyl, insbesondere für Phenyl und m für 1, 2 oder 3, insbesondere 1 oder 2, bevorzugt für 1 steht, anwenden.

Bei den Ausgangsverbindungen kann es sich um Brom oder Chlor-Verbindungen handeln, wie beispielsweise:
4-Chlor-1,2-difluorbenzol, 3-Chlor-1,2-difluorbenzol, 2-Chlor-1,4-difluorbenzol, 5-Chlor-1,2,4-trifluorbenzol, 3-Chlor-1,2,4-trifluorbenzol, 3-Chlor-1,2,5-trifluorbenzol, 4-Chlor-1,2,3-trifluorbenzol, 5-Chlor-1,2,3-trifluorbenzol, 4-Brom-1,2-difluorbenzol, 3-Brom-1,2-difluorbenzol, 2-Brom-1,4-difluorbenzol, 5-Brom-1,2,4-trifluorbenzol, 3-Brom-1,2,4-trifluorbenzol, 3-Brom-1,2,5-trifluorbenzol, 4-Brom-1,2,3-trifluorbenzol, 5-Brom-1,2,3-trifluorbenzol oder 5-Chlor-2,3-difluorpyridin. In das Verfahren können auch Gemische von Verbindungen der Formel (II) eingesetzt werden, die nach Umsetzung eine einheitliche Verbindung der Formel (I) ergeben. Dabei können diese Verbindungen mit gleichem Substitutionsmuster bezüglich der Endverbindung auch als Mischungen verschiedener Chlor- und Bromverbindungen eingesetzt werden.

Besonders vorteilhaft lassen sich nach diesem Verfahren 1,2,4-Trifluorbenzol, 1,2,3-Trifluorbenzol, 1,2-Difluorbenzol, 1,4-Difluorbenzol, 2,3-Difluorpyridin herstellen.

Es ist zweckmäßig den Katalysator auf einem Trägermaterial, wie beispielsweise Aktivkohle, Calciumcarbonat, Bariumsulfat, Bimsstein, Tonerde, Kieselgur, Kieselgel und/oder Aluminiumoxid anzuwenden. Bevorzugt wird Palladium auf Aktivkohle oder Aluminiumoxyd als Trägermaterial gebracht.

Der Palladiumgehalt des Trägerkatalysators liegt bevorzugt bei 0,1 - 10 Gew.-%, vorzugsweise bei 0,2 bis 8 Gew.-%, besonders bevorzugt bei 0,5 - 6 Gew.-% Palladium, bezogen auf den gesamten Katalysator.

Die Menge des benötigten Katalysators liegt im Bereich von 0,01 - 50 mmol Palladium bezogen auf die Äquivalente abzuspaltendes Halogen (Chlor/Brom).

Der Katalysator kann problemlos recylisiert, d.h. für mehrere Halogenspaltungesprozesse verwendet werden.

An Aminen können Monoamine oder Polyamine mit zwei bis vier Aminogruppen oder Gemische daraus dienen, mit der Eigenschaft, daß sowohl die freie Base als auch das mit der entstehenden HX gebildete Basenhydrohalogenid unter den Reaktions- und Aufarbeitungsbedingungen nicht wasserlöslich sind.

In vielen Fällen haben sich Alkylamine als günstig erwiesen.

Besonders geeignet sind Amine der allgemeinen Formel (III)

$$H_pN(C_rH_{2r+1})_q \qquad (III)$$

mit p = 0, 1 oder 2; q = 1, 2 oder 3 und p + q = 3 ; r = 5 bis 20, bevorzugt 8 bis 15, wobei die Alkylreste gleich oder ungleich, verzweigt oder unverzweigt sein können. Insbesondere steht p für 0 oder 1 und q für 2 oder 3.

Hochwirksame aliphatische Amine sind im einzelnen Tri-(n-dodecyl)-amin; Tri-(iso-oktyl)-amin; Trialkyl-(C8/C10)-amine oder Mischungen aus ihnen.

Obwohl die vorstehend genannten Trialkylamine der genannten Formel (III) am geeignetsten sind, können prinzipiell auch Arylamine oder Aralkylamine eingesetzt werden.

Sehr gute Ergebnisse werden erhalten, wenn sowohl die eingesetzten Amine als auch die entstehenden Hydrohalogenide flüssig sind.

Es hat sich in vielen Fällen bewährt mit Aminkonzentration von 50 bis 500 Mol% Amin pro Äquivalent abzuspaltendes Halogen zu arbeiten; insbesondere wird das Amin in Mengen von 80 bis 250 Mol%, bevorzugt 100 bis 150 Mol% pro Äquivalent abzuspaltendes Halogen eingesetzt.

Falls es sich bei den verwendeten Edukten und Produkten um bei Reaktions-und Aufarbeitungstemperatur flüssige Verbindungen handelt, hat es sich als günstig erwiesen ohne ein zusätzliches Lösungsmittel zu arbeiten. Bei festen Produkten kann in Gegenwart eines inerten Lösungsmittels wie beispielsweise Benzol, Toluol, Xylol, Alkanole (C1-C4): Methanol, Ethanol Propanol, Polyglykole: Ethylenglykol, Dialkylether: Diethylether, Methylethylether, Tetrahydrofuran, Pentan, Hexan, Heptan, Polyether: Polyethylenglykoldimethylether 500 oder Mischungen dieser Lösungsmittel gearbeitet werden.

Die Anwesenheit von Wasser ist in diesem Verfahren nicht erforderlich. Es kann in Gegenwart von Wasser umgesetzt werden, es ist jedoch vorteilhaft mit einem möglichst geringen Wassergehalt, d.h. kleiner 5 Gew.-%, insbesondere kleiner 1 Gew.-% bezogen auf die gesamte Reaktionslösung, zu arbeiten.

Das Verfahren kann sowohl bei Atmosphärendruck als auch bei Überdruck durchgeführt werden. Es ist zweckmäßig, bei einem Wasserstoffüberdruck von 0,1 bis 50 bar umzusetzen.

Es hat sich in vielen Fällen bewährt, das Verfahren bei Temperaturen von 0° bis 150°C, insbesondere 40°C bis 120°C durchzuführen. Die Anwendung zu tiefer Temperaturen führt dabei zu einer langsamen und unvollständigen Reaktion. Zu hoch gewählte Temperaturen können zum Teil unerwünschte Fluorabspaltung zur Folge haben.

Das am Ende der Reaktion gebildete Aminhydrohalogenid kann auf einfache und vorteilhafte Weise regeneriert werden, indem die Rohlösung mit wäßriger Base behandelt wird. Dabei bildet sich praktisch ohne Verluste das freie Amin, das in der Folgereaktion nach Abtrennen des Produktes, ohne weitere Vorbehandlung wieder eingesetzt werden kann.

Durch exakte Neutralisation des Aminhydrohalogenids wird nur soviel Base verbraucht, als Äquivalente Fluoraromat entstanden sind. Das entstehende Abwasser reagiert neutral.

Der bei der Reaktion anfallende gebrauchte Katalysator kann unbehandelt weiterverwendet, oder durch bekannte Reinigungsverfahren wie beispielsweise durch Wasserdampf gereinigt werden.

Ein besonderer Vorteil des erfindungsgemäßen Verfahrens besteht darin, daß bei der Reaktion praktisch keinerlei Nebenprodukte gebildet werden, so daß verbleibende Mutterlaugen, Vorläufe, Zwischenläufe und Destillationsrückstände ohne Gefahr von Verunreinigung in den Folgeansätzen zurückgeführt werden können. Hierdurch ist das Verfahren ökologisch und ökonomisch außerordentlich vorteilhaft.

Die Ausgangsverbindungen des erfindungsgemäßen Verfahrens können durch Nitrierung der entsprechenden Chlorfluorbenzole bzw. durch Chlorfluoraustausch-Reaktionen an Chlornitroaromaten hergestellt werden. Werden als Ausgangsverbindung Chlorfluoraromaten benötigt, lassen sich diese auf einfache Weise durch eine denitrierende Chlorierung der entsprechenden Fluornitroaromaten herstellen.

Die folgenden Beispiele dienen zur Erläuterung des erfindungsgemäßen Verfahrens ohne sich darauf zu beschränken.

Beispiel 1

Zur Herstellung von 1,2,3-Trifluorbenzol werden 299,7 g 1,8 mol 4-Chlor-1,2,3-trifluorbenzol, 4,1 g Pd/C (5%-ig, 50% wasserfeucht) als Katalysator, zusammen mit 846,3 g 2,12 mol Tri-(C8/C10)alkylamin als Base im Reaktionsgefäß (Autoklav) vorgelegt. Die Reaktionslösung wird auf 75°C aufgeheizt und bei dieser Temperatur mit Wasserstoff reduktiv entchloriert. Nach beendeter Wasserstoffaufnahme wird kurz nachgerührt, auf Raumtemperatur gekühlt, die Reaktionslösung mit Natronlauge neutralgestellt und der Katalysator vom Reaktionsgemisch abgenutscht. Nach dem Abtrennen der organischen Phase wird diese bei Normaldruck andestilliert, das erhaltene Destillat getrocknet und anschließend fraktioniert. Verbleibende Mutterlauge, Vorläufe, Zwischenläufe und Destillationsrückstände werden ohne weitere Vorbehandlung in den Folgeansatz zurückgeführt.

Umsatz:     95,1 % (nach GC)
Ausbeute:    219,2 g (1,66 mol) 1,2,3-Trifluorbenzol 92,2 % bezogen auf eingesetztes 4-Chlor-1,2,3-trifluorbenzol.
Reinheit:    >99,9 (GC-Flächen-%) 1,2,3-Trifluorbenzol

Folgeansatz

Die Aminmutterlauge wird zusammen mit den Produktionsrückständen des Startansatzes mit 299,7 g 1,8 mol 4-Chlor-1,2,3-trifluorbenzol und 4,1 g Pd/C (5%-ig, 50% wasserfeucht) aufgestärkt und analog dem Startansatz reduktiv entchloriert und entsprechend aufgearbeitet.

Die aus der Fraktionierung resultierenden Produktionsrückstände (Vorläufe, Zwischenläufe und Destillationsrückstände) werden ohne weitere Vorbehandlung in den Folgeansatz zurückgeführt.

Umsatz:     98,1 % (nach GC)
Ausbeute:    231,0 g (1,75 mol) 1,2,3-Trifluorbenzol 97,2 % bezogen auf eingesetztes 4-Chlor-1,2,3-trifluorbenzol.
Reinheit:    >99,9 (GC-Flächen-%) 1,2,3-Trifluorbenzol

Beispiel 2

Zur Herstellung von 1,2,4-Trifluorbenzol werden 299,7 g 1,8 mol 5-Chlor-1,2,4-trifluorbenzol, 4,1 g Pd/C (5%-ig, 50% wasserfeucht) als Katalysator, zusammen mit 846,3 g 2,12 mol Tri-(G8/C1 0)alkylamin als Base im Reaktionsgefäß (Autoklav) vorgelegt. Die Reaktionslösung wird auf 90°C aufgeheizt und bei dieser Temperatur mit Wasserstoff reduktiv entchloriert. Nach beendeter Wasserstoffaufnahme wird kurz nachgerührt, auf Raumtemperatur gekühlt, die Reaktionslösung mit Natronlauge neutralgestellt und der Katalysator vom Reaktionsgemisch abgenutscht. Nach dem Abtrennen der organische Phase wird diese bei Normaldruck andestilliert, das erhaltene Destillat getrocknet und anschließend fraktioniert. Verbleibende Mutterlauge, Vorläufe, Zwischenläufe und Destillationsrückstände werden ohne Vorbehandlung in den Folgeansatz zurückgeführt.

Umsatz:     93,0 % (nach GG)
Ausbeute:    214,7 g (1,63 mol) 1,2,4-Trifluorbenzol 90,3 % bezogen auf eingesetztes 5-Chlor-1,2,4-trifluorbenzol.
Reinheit:    >99,9 (GC-Flächen-%) 1,2,4-Trifluorbenzol

Folgeansatz

Die Aminmutterlauge wird zusammen mit den Produktionsrückständen des Startansatzes mit 299,7 g 1,8 mol 4-Chlor-1,2,4-trifluorbenzol und 4,1 g Pd/C (5%-ig, 50% wasserfeucht) aufgestärkt und analog dem Startansatz reduktiv entchloriert und entsprechend aufgearbeitet.

Die aus der Fraktionierung resultierenden Produktionsrückstände (Vorläufe, Zwischenläufe und Destillationsrückstände) werden ohne weitere Vorbehandlung in den Folgeansatz zurückgeführt.

Umsatz:     98,0 % (nach GC)
Ausbeute:    228,1 g (1,72 mol) 1,2,4-Trifluorbenzol 96,0 % bezogen auf eingesetztes 4-Chlor-1,2,4-trifluorbenzol.
Reinheit:    >99,9 (GC-Flächen-%) 1,2,4-Trifluorbenzol

Beispiel 3

Zur Herstellung von 1,2-Difluorbenzol werden 360 g 2,2 mol einer Mischung aus 47% Chlor-1,2-difluorbenzolen (zwei Isomere im Verhältnis 1 : 3,5) und 52% Dichlor-1,2-difluorbenzolen (zwei Isomere im Verhältnis 4,3 : 0,9), 12,5g Pd/C (5%-ig, 50% wasserfeucht) als Katalysator, zusammen mit 1037,9 g 2,6 mol Tri-(C8/C10)alkylamin als Base im Reaktionsgefäß (Autoklav) vorgelegt. Die Reaktionslösung wird auf 65°C aufgeheizt und bei dieser Temperatur mit Wasserstoff reduktiv entchloriert. Nach beendeter Wasserstoffaufnahme wird kurz nachgerührt, auf Raumtemperatur gekühlt, die Reaktionslösung mit Natronlauge neutralgestellt und der Katalysator vom Reaktionsgemisch abgenutscht. Nach dem Abtrennen der organische Phase wird diese bei Normaldruck andestilliert, das erhaltene Destillat getrocknet und anschließend fraktioniert. Verbleibende Mutterlauge, Vorläufe, Zwischenläufe und Destillationsrückstände können in Folgeansätze zurückgeführt werden.

Umsatz:     98,0 % (nach GG)
Ausbeute:    211,7g (1,86 mol) 1,2-Difluorbenzol 84,5 % bezogen auf eingesetzte Mischung von Chlor-und Dichlor-1,2-difluorbenzolen.
Reinheit:    >99,2 (GC-Flächen-%) 1,2-Difluorbenzol

Beispiel 4

Zur Herstellung von 2,3-Difluorpyridin werden 179,4 g (1,2 mol) 5-Chlor-2,3-difluorpyridin, 4,5 g Pd/C (5 %-ig, 50% wasserfeucht) als Katalysator, zusammen mit 598,5 g 1,5 mol Tri-(C8/C10)alkylamin als Base im Reaktionsgefäß (Autoklav) vorgelegt. Die Reaktionslösung wird auf 90°C aufgeheizt und bei dieser Temperatur mit Wasserstoff reduktiv entchloriert. Nach beendeter Wasserstoffaufnahme wird kurz nachgerührt, auf Raumtemperatur gekühlt, die Reaktionslösung mit Natronlauge neutralgestellt und der Katalysator vom Reaktionsgemisch abgenutscht. Nach dem Abtrennen der organische Phase wird diese bei Normaldruck andestilliert, das erhaltene Destillat getrocknet und anschließend fraktioniert. Verbleibende Mutterlauge, Vorläufe, Zwischenläufe und Destillationsrückstände werden ohne weitere Vorbehandlung in den Folgeansatz zurickgeführt.

Umsatz:    95,0 % (nach GG)
Ausbeute:   127,7 g (1,1 mol) 2,3-Difluorpyridin 91,7 % bezogen auf eingesetztes 5-Chlor-2,3-difluorpyridin.
Reinheit:    >99 (GC-Flächen-%) 2,3-Difluorpyridin

Folgeansatz

Die Aminmutterlauge wird zusammen mit den Produktionsrückständen des Startansatzes mit 179.4 g (1,2 mol) 5-Chlor-2,3-difluorpyridin, 4,5 g Pd/C (5%-ig, 50% wasserfeucht) aufgestärkt und analog dem Startansatz reduktiv entchloriert und entsprechend aufgearbeitet.

Die aus der Fraktionierung resultierenden Produktionsrückstände (Vorläufe, Zwischenläufe und Destillationsrückstände) werden ohne weitere Vorbehandlung in den Folgeansatz zurückgeführt.

Umsatz:    99,5 % (nach GC)

Ausbeute:   135,6 g (1,18 mol) 2,3-Difluorpyridin 98,2 % bezogen auf eingesetztes 5-Chlor-2,3-difluorpyridin.

Reinheit:    > 99,9 (GC-Flächen-%) 2,3-Difluorpyridin

## Patentansprüche

1.   Verfahren zur Herstellung von Fluoraromaten der Formel I - ausgenommen 1,3-Difluorbenzol -

$$F_nArR^1R^2R^3 \qquad (I)$$

worin

Ar Phenyl, Naphthyl, Pyridyl, $R^1$, $R^2$, $R^3$ unabhängig voneinander Wasserstoff, $(C_1-C_4)$-Alkyl, Phenyl, $NR_2$, OR, CN, COH, COR, wobei R Wasserstoff, $(C_1-C_6)$-Alkyl und n = 1, 2, 3, 4 oder 5

bedeuten, dadurch gekennzeichnet, daß man Fluoraromaten der Formel II

$$X_mF_nArR^1R^2R^3 \qquad (II)$$

worin Ar, $R^1$, $R^2$, $R^3$ und n die oben erwähnte Bedeutung besitzen,
X für Chlor- oder Bromatome steht und
m = 1, 2, 3, 4 oder 5 bedeutet, in Gegenwart eines Palladium-Katalysators, eines nicht wasserlöslichen Amins, das ebenfalls nicht wasserlösliche Hydrohalogenide bildet und gegebenenfalls eines inerten Lösungsmittels mit Wasserstoff umsetzt.

2.   Verfahren nach Anspruch 1, dadurch gekennzeichnet, daß als geeignete Fluoraromaten der Formel (II)
4-Chlor-1,2-difluorbenzol, 3-Chlor-1,2-difluorbenzol, 2-Chlor-1,4-difluorbenzol, 5-Chlor-1,2,4-trifluorbenzol, 3-Chlor-1,2,4-trifluorbenzol, 3-Chlor-1,2,5-trifluorbenzol, 4-Chlor-1,2,3-trifluorbenzol, 5-Chlor-1,2,3-trifluorbenzol, 4-Brom-1,2-difluorbenzol, 3-Brom-1,2-difluorbenzol, 2-Brom-1,4-difluorbenzol, 5-Brom-1,2,4-trifluorbenzol, 3-Brom-1,2,4-trifluorbenzol, 3-Brom-1,2,5-trifluorbenzol, 4-Brom-1,2,3-trifluorbenzol, 5-Brom-1,2,3-trifluorbenzol oder 5-Chlor-2,3-difluorpyridin verwendet werden.

3.   Verfahren nach Anspruch 1, dadurch gekennzeichnet, daß Gemische von Verbindungen der Formel (II) eingesetzt werden, die nach Umsetzung eine einheitliche Verbindung der Formel (I) ergeben.

4.   Verfahren nach mindestens einem der Ansprüche 1 bis 3, dadurch gekennzeichnet, daß 1,2,4-Trifluorbenzol, 1,2,3-Trifluorbenzol, 1,2-Difluorbenzol, 1,4-Difluorbenzol, 2,3-Difluorpyridin hergestellt werden.

5.   Verfahren nach mindestens einem der Ansprüche 1 bis 4, dadurch gekennzeichnet, daß bei Temperaturen von 0 bis 150°C, insbesondere 40° bis 120°C umgesetzt wird.

6.   Verfahren nach mindestens einem der Ansprüche 1 bis 4, dadurch gekennzeichnet, daß in der Reaktionslösung die Wassermenge kleiner 5 Gew.-%, insbesondere kleiner 1 Gew.-%, bezogen auf die gesamte Reaktionslösung, ist.

7.   Verfahren nach mindestens einem der Ansprüche 1 bis 5, dadurch gekennzeichnet, daß als Palladium-Katalysator ein Palladium-Katalysator auf einem Trägermaterial eingesetzt wird.

8.   Verfahren nach Anspruch 7, dadurch gekennzeichnet, daß als Trägermaterial Aktivkohle, Calciumcarbonat, Bariumsulfat, Bimsstein, Tonerde, Kieselgur, Kieselgel und/oder Aluminiumoxid, insbesondere Aktivkohle oder Aluminiumoxid einge-

**9.** Verfahren nach Anspruch 7 oder 8, dadurch gekennzeichnet, daß der Katalysator 0,1 - 10 Gew.-%, insbesondere 0,2 - 8 Gew.-%, bevorzugt 0,5 - 6 Gew.-% Palladium, bezogen auf das verwendete Trägermaterial, enthält.

**10.** Verfahren nach mindestens einem der Ansprüche 1 bis 9, dadurch gekennzeichnet, daß als Katalysator 0,01 bis 50 mmol Palladium, bezogen auf Äquivalente abzuspaltendes Halogen, eingesetzt werden.

**11.** Verfahren nach mindestens einem der Ansprüche 1 bis 10, dadurch gekennzeichnet, daß der Katalysator recyclisiert wird.

**12.** Verfahren nach mindestens einem der Ansprüche 1 bis 11, dadurch gekennzeichnet, daß als Amine Alkylamine eingesetzt werden.

**13.** Verfahren nach mindestens einem der Ansprüche 1 bis 12, dadurch gekennzeichnet, daß als Amin ein Amin der allgemeinen Formel (III)

$$H_pN(C_rH_{2r+1})_q \qquad (III)$$

worin $p = 0, 1$ oder 2; $q = 1, 2$ oder 3; $p + q = 3$ und $r = 5$ bis 20, insbesondere 8 bis 15 ist, wobei die Alkylreste gleich oder ungleich, verzweigt oder unverzweigt sein können, eingesetzt wird.

**14.** Verfahren nach mindestens einem der Ansprüche 1 bis 13, dadurch gekennzeichnet, daß als Amine Tri-(n-dodecyl)-amin, Tri-(iso-oktyl)-amin, Trialkyl-($C_8/C_{10}$)-amine oder Mischungen dieser Amine verwendet werden.

**15.** Verfahren nach mindestens einem der Ansprüche 1 bis 14, dadurch gekennzeichnet, daß die verwendeten aliphatischen Amine bei Reaktions- und Aufarbeitungstemperatur im Reaktionsmedium flüssig sind.

**16.** Verfahren nach mindestens einem der Ansprüche 1 bis 15, dadurch gekennzeichnet, daß die aus den verwendeten aliphatischen Aminen entstehenden Hydrohalogenide im Reaktionsmedium flüssig sind.

**17.** Verfahren nach mindestens einem der Ansprüche 1 bis 16, dadurch gekennzeichnet, daß das Alkylamin in Mengen von 50 bis 500 Mol%, insbesondere 80 bis 250 Mol%, bevorzugt 100 bis 150 Mol%, bezogen auf Äquivalente abzuspaltendes Halogen, verwendet wird.

**18.** Verfahren nach mindestens einem der Ansprüche 1 bis 14, dadurch gekennzeichnet, daß bei Normal- oder Überdruck, insbesondere bei einem Wasserstoffüberdruck von 0,1 bis 50 bar umgesetzt wird.

**19.** Verfahren nach mindestens einem der Ansprüche 1 bis 18, dadurch gekennzeichnet, daß als Lösungsmittel Benzol, Toluol, Xylol, Alkanole, ($C_1$-$C_4$): Methanol, Ethanol, Propanol; Polyglykole: Ethylenglykol; Dialkylether: Diethylether, Methylethylether, Tetrahydrofuran, Pentan, Hexan, Heptan; Polyether: Polyethylenglykoldimethylether 500 oder Mischungen dieser Lösungsmittel eingesetzt werden.

**20.** Verfahren nach mindestens einem der Ansprüche 1 bis 19, dadurch gekennzeichnet, daß das Amin recyclisiert wird.

**21.** Verfahren nach mindestens einem der Ansprüche 1 bis 19, dadurch gekennzeichnet, daß alle Produktionsrückstände in den Folgeansatz zurückgeführt werden.

**Claims**

**1.** A process for the preparation of aromatic fluoro compounds of the formula I - with the exception of 1,3-difluorobenzene -

$$F_nArR^1R^2R^3 \qquad (I)$$

in which

Ar is phenyl, naphthyl or pyridyl, $R^1$, $R^2$ and $R^3$ independently of one another are hydrogen, ($C_1$-$C_4$)-alkyl, phenyl, $NR_2$, OR, CN, COH or COR, where R is hydrogen or ($C_1$-$C_6$)-alkyl, and $n = 1, 2, 3, 4$ or 5,

which comprises reacting aromatic fluoro compounds of the formula II

$$X_mF_nArR^1R^2R^3 \qquad (II)$$

in which Ar, $R^1$, $R^2$, $R^3$ and n have the abovementioned meaning,
each X is a chlorine or bromine atom and
$m = 1, 2, 3, 4$ or 5, with hydrogen in the presence of a palladium catalyst, a water-insoluble amine which also does not form water-soluble hydrohalides, and if desired an inert solvent.

**2.** The process as claimed in claim 1, which comprises using
4-chloro-1,2-difluorobenzene, 3-chloro-1,2-difluorobenzene, 2-chloro-1,4-difluorobenzene, 5-chloro-1,2,4-trifluorobenzene, 3-chloro-1,2,4-trifluorobenzene, 3-chloro-1,2,5-trifluorobenzene, 4-chloro-1,2,3-trifluorobenzene, 5-chloro-1,2,3-trifluoroben-

EP 0 667 328 B1

zene, 4-bromo-1,2-difluorobenzene, 3-bromo-1,2-difluorobenzene, 2-bromo-1,4-difluorobenzene, 5-bromo-1,2,4-trifluorobenzene, 3-bromo-1,2,4-trifluorobenzene, 3-bromo-1,2,5-trifluorobenzene, 4-bromo-1,2,3-trifluorobenzene, 5-bromo-1,2,3-trifluorobenzene or 5-chloro-2,3-difluoropyridine as suitable aromatic fluoro compounds of the formula (II).

3. The process as claimed in claim 1, which comprises employing mixtures of compounds of the formula (II) which result, after reaction, in a uniform compound of the formula (I).

4. The process as claimed in at least one of claims 1 to 3, which comprises preparing 1,2,4-trifluorobenzene, 1,2,3-trifluorobenzene, 1,2-difluorobenzene, 1,4-difluorobenzene or 2,3-difluoropyridine.

5. The process as claimed in at least one of claims 1 to 4, which is carried out at temperatures of from 0 to 150°C, in particular from 40° to 120°C.

6. The process as claimed in at least one of claims 1 to 4, wherein the quantity of water in the reaction solution is less than 5 % by weight and in particular less than 1 % by weight, based on the overall reaction solution.

7. The process as claimed in at least one of claims 1 to 5, wherein the palladium catalyst is employed on a support material.

8. The process as claimed in claim 7, wherein the support material employed is activated charcoal, calcium carbonate, barium sulfate, pumice, argillaceous earth, kieselguhr, silica gel and/or alumina, especially activated charcoal or alumina.

9. The process as claimed in claim 7 or 8, wherein the catalyst contains 0.1 - 10 % by weight, in particular 0.2 - 8 % by weight and preferably 0.5 - 6 % by weight of palladium, based on the support material used.

10. The process as claimed in at least one of claims 1 to 9, wherein the catalyst employed is from 0.01 to 50 mmol of palladium, based on equivalents of halogen to be eliminated.

11. The process as claimed in at least one of claims 1 to 10, wherein the catalyst is recycled.

12. The process as claimed in at least one of claims 1 to 11, wherein the amines employed are alkylamines.

13. The process as claimed in at least one of claims 1 to 12, wherein the amine employed is an amine of the formula (III)

$$H_pN(C_rH_{2r+1})_q \qquad (III)$$

where p = 0, 1 or 2; q = 1, 2 or 3; p + q = 3 and r = 5 to 20, in particular 8 to 15, and the alkyl radicals may be identical or different and branched or unbranched.

14. The process as claimed in at least one of claims 1 to 13, wherein the amines used are tri-(n-dodecyl)amine, tri(iso-octyl)amine, tri-alkyl($C_8/C_{10}$)amines or mixtures of these amines.

15. The process as claimed in at least one of claims 1 to 14, wherein the aliphatic amines used are liquid in the reaction medium at reaction and work-up temperature.

16. The process as claimed in at least one of claims 1 to 15, wherein the hydrohalides resulting from the aliphatic amines used are liquid in the reaction medium.

17. The process as claimed in at least one of claims 1 to 16, wherein the alkylamine is used in quantities of from 50 to 500 mol%, in particular from 80 to 250 mol% and preferably from 100 to 150 mol%, based on equivalents of halogen to be eliminated.

18. The process as claimed in at least one of claims 1 to 14, which is carried out at atmospheric or superatmospheric pressure, in particular at a hydrogen overpressure of from 0.1 to 50 bar.

19. The process as claimed in at least one of claims 1 to 18, wherein the solvents employed are benzene, toluene, xylene, ($C_1$-$C_4$)-alkanols: methanol, ethanol or propanol; polyglycols: ethylene glycol; dialkyl ethers: diethyl ether or methyl ethyl ether, tetrahydrofuran, pentane, hexane or heptane; polyethers: polyethylene glycol dimethyl ether 500, or mixtures of these solvents.

20. The process as claimed in at least one of claims 1 to 19, wherein the amine is recycled.

21. The process as claimed in at least one of claims 1 to 19, wherein all the production residues are recycled to the subsequent batch.

**Revendications**

1. Procédé pour la préparation de composés aromatiques fluorés (exception faite du 1,3-difluorobenzène) de formule I

$$F_nArR^1R^2R^3 \qquad (I)$$

où Ar représente un phényle, un naphtyle, un pyridyle, $R^1$, $R^2$, $R^3$ représentent, indépendamment chacun l'un de l'autre, un atome d'hydrogène, , un alkyle en $C_1$-$C_4$, un phényle, $NR_2$, OR, CN, COH, COR, où R représente un atome d'hydrogène, un alkyle en $C_1$-$C_6$ et n = 1, 2, 3, 4 ou 5,

caractérisé en ce que l'on fait réagir des composés aromatiques fluorés de formule (II)

$$X_mF_nArR^1R^2R^3 \qquad (II)$$

où Ar, $R^1$, $R^2$, $R^3$ et n possèdent les significations citées ci-dessus,
X représente des atomes de chlore ou de brome et
m vaut 1, 2, 3, 4 ou 5,
en présence d'un catalyseur au palladium, d'une amine non soluble dans l'eau, qui forme un halogénure d'hydrogène non soluble dans l'eau et éventuellement d'un solvant inerte, avec de l'hydrogène.

2. Procédé selon la revendication 1, caractérisé en ce que l'on utilise comme composés aromatiques fluorés appropriés de formule (II) :
le 4-chloro-1,2-difluorobenzène, le 3-chloro-1,2-difluorobenzène,
le 2-chloro-1,4-difluorobenzène, le 5-chloro-1,2,4-trifluorobenzène,
le 3-chloro-1,2,4-trifluorobenzène, le 3-chloro-1,2,5-trifluorobenzène,
le 4-chloro-1,2,3-trifluorobenzène, le 5-chloro-1,2,3-trifluorobenzène,
le 4-bromo-1,2-difluorobenzène, le 3-bromo-1,2-difluorobenzène,
le 2-bromo-1,4-difluorobenzène, le 5-bromo-1,2,4-trifluorobenzène,
le 3-bromo-1,2,4-trifluorobenzène, le 3-bromo-1,2,5-trifluorobenzène,
le 4-bromo-1,2,3-trifluorobenzène, le 5-bromo-1,2,3-trifluorobenzène ou
la 5-chloro-2,3-difluoropyridine.

3. Procédé selon la revendication 1, caractérisé en ce qu'on utilise des mélanges de composés de formule (II) qui donnent, après réaction, un composé homogène de formule (I).

4. Procédé selon au moins l'une des revendications 1 à 3, caractérisé en ce que l'on prépare le 1,2,4-trifluorobenzène, le 1,2,3-trifluorobenzène, le 1,2-difluorobenzène, le 1,4-difluorobenzène, la 2,3-difluoropyridine.

5. Procédé selon au moins l'une des revendications 1 à 4, caractérisé en ce que l'on réalise la réaction à des températures de 0 °C à 150 °C, en particulier de 40 °C à 120 °C.

6. Procédé selon au moins l'une des revendications 1 à 4, caractérisé en ce que l'on utilise dans la solution réactionnelle des quantités d'eau inférieures à 5 % en poids, en particulier inférieures à 1 % en poids, par rapport à la totalité de la solution réactionnelle.

7. Procédé selon au moins l'une des revendications 1 à 5, caractérisé en ce que l'on utilise comme catalyseur au palladium un catalyseur au palladium sur un support.

8. Procédé selon la revendication 7, caractérisé en ce que l'on utilise comme support du charbon actif, du carbonate de calcium, du sulfate de baryum, de la pierre-ponce argile du kieselgur, du gel de silice et/ou de l'oxyde d'aluminium, en particulier du charbon actif ou de l'oxyde d'aluminium.

9. Procédé selon la revendication 7 ou 8, caractérisé en ce que le catalyseur contient de 0,1 à 10 % en poids, en particulier de 0,2 à 8 % en poids, de préférence de 0,5 à 6 % de palladium, par rapport au support utilisé.

10. Procédé selon au moins l'une des revendications 1 à 9, caractérisé en ce que l'on utilise comme catalyseur de 0,01 à 50 mmoles de palladium, par rapport aux équivalents d'halogène à dissocier.

11. Procédé selon au moins l'une des revendications 1 à 10, caractérisé en ce que l'on peut recycler le catalyseur.

12. Procédé selon au moins l'une des revendications 1 à 11, caractérisé en ce que l'on utilise des alkylamines comme amines.

13. Procédé selon au moins l'une des revendications 1 à 12, caractérisé en ce que l'on utilise comme amine une amine de formule générale (III)

$$H_pN(C_rH_{2r+1})_q \qquad (III)$$

avec p = 0, 1 ou 2 ; q = 1, 2 ou 3, et p + q = 3 et r = de 5 à 20, en particulier de 8 à 15, les restes alkyle pouvant être identiques ou différents, ramifiés ou non ramifiés.

14. Procédé selon au moins l'une des revendications 1 à 13, caractérisé en ce que l'on utilise comme amines la tri-(n-dodécyl)amine, la tri(iso-octyl)amine, des trialkyl($C_8$/$C_{10}$)amines ou des mélanges de ces amines.

15. Procédé selon au moins l'une des revendications 1 à 14, caractérisé en ce que les amines aliphatiques utilisées sont liquides dans le milieu réactionnel à la température réactionnelle et à la temperature de préparation.

16. Procédé selon au moins l'une des revendications 1 à 15, caractérisé en ce que les hydrohalogénures formés à partir des amines aliphatiques utilisées sont liquides dans le milieu réactionnel.

17. Procédé selon au moins l'une des revendications 1 à 16, caractérisé en ce que l'on utilise l'alkylamine en des quantités de 50 à 500 % en mole, en particulier de 80 à 250 % en mole, de préférence de 100 à 150 % en mole, par rapport aux équivalents d'halogène à dissocier.

18. Procédé selon au moins l'une des revendications 1 à 14, caractérisé en ce que l'on met en oeuvre la réaction à la pression normale ou à une surpression, en particulier à une surpression d'hydrogène de 0,1 à 50 bars.

19. Procédé selon au moins l'une des revendications 1 à 18, caractérisé en ce que l'on utilise comme solvant le benzène, le toluène, le xylène, des alcanols en $C_1$-$C_4$ : le méthanol, l'éthanol, le propanol; des polyglycols : l'éthylèneglycol; des dialkyléthers : le diéthyléther, le méthyléthyléther, le tétrahydrofuranne, le pentane, l'hexane, l'heptane; des polyéthers : le diméthyléther de polyéthylèneglycol 500 ou des mélanges de ces solvants.

20. Procédé selon au moins l'une des revendications 1 à 19, caractérisé en ce que l'on peut recycler l'amine.

21. Procédé selon au moins l'une des revendications 1 à 19, caractérisé en ce que l'on recycle les résidus des produits dans la charge suivante.